(19) Europäisches Patentamt
European Patent Office
Office européen des brevets

(11) Veröffentlichungsnummer: **0 183 976**
**B1**

(12) # EUROPÄISCHE PATENTSCHRIFT

(45) Veröffentlichungstag der Patentschrift: **08.06.88**

(21) Anmeldenummer: **85113672.1**

(22) Anmeldetag: **28.10.85**

(51) Int. Cl.⁴: **C 07 C 119/042,** C 07 C 119/045, C 07 C 118/00, C 07 C 127/24, C 07 D 251/34

(54) **Verfahren zur Herstellung von wärmefarbbeständigen aliphatischen und/oder cycloaliphatischen Diisocyanaten und ihre Verwendung zur Herstellung von farbverbesserten, modifizierten Polyisocyanaten.**

(30) Priorität: **09.11.84 DE 3440912**

(43) Veröffentlichungstag der Anmeldung: **11.06.86 Patentblatt 86/24**

(45) Bekanntmachung des Hinweises auf die Patenterteilung: **08.06.88 Patentblatt 88/23**

(84) Benannte Vertragsstaaten: **DE FR GB IT**

(56) Entgegenhaltungen:
**DE-A-1 568 623**
**FR-A-2 184 592**
**FR-A-2 273 799**
**FR-A-2 489 339**
**US-A-3 183 112**
**US-A-3 873 589**
**US-A-3 903 127**

(73) Patentinhaber: **BAYER AG, Konzernverwaltung RP Patentabteilung, D-5090 Leverkusen 1 Bayerwerk (DE)**

(72) Erfinder: **Müller, Hanns Peter, Dr., Im Kerberich 6, D-5068 Odenthal (DE)**
Erfinder: **Clarenz, Werner, Dr., Wagnerstrasse 21, D-5060 Bergisch Gladbach 2 (DE)**
Erfinder: **Grave, Heinrich, Dr., Haydn- Strasse 4b, D-5060 Bergisch Gladbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 183 976 B1

LIBER, STOCKHOLM 1988

**Beschreibung**

Die Erfindiung betrifft ein neues Verfahren zur Verbesserung der Wärmefarbbeständigkeit von aliphatischen und/oder cycloaliphatischen Diisocyanaten und die Verwendung der Verfahrensprodukte zur Herstellung von modifizierten Polyisocyanaten, insbesondere von Biuretgruppen aufweisenden Polyisocyanaten einer verringerten Eigenfarbe.

Organische Polyisocyanate, insbesondere solche mit aliphatisch und/oder cycloaliphatisch gebundenen Isocyanatgruppen, finden auf dem Gebiet der Herstellung von lichtbeständigen Polyurethankunststoffen und Lacküberzügen höchster Lichtechtheit, hoher Kreidungsbeständigkeit und ausgezeichneter Glanzhaltung eine ausgedehnte praktische Verwendung.

Für diese Einsatzgebiete werden in der Praxis in der Regel Modifizierungsprodukte einfacher aliphatischer bzw. cycloaliphatischer Diisocyanate mit einem verringertem Dampfdruck eingesetzt. Derartige Modifizierungsprodukte sind beispielsweise Allophanat-, Uretdion-, Harnstoff-, Semicarbazid-, Urethan- und insbesondere Biuret- oder Isocyanuratstruktureinheiten aufweisende Polyisocyanate eines verminderten Dampfdrucks. Bei der technischen Herstellung dieser Modifizierungsprodukte aus den entsprechenden einfachen Diisocyanaten führt oftmals die nicht ausreichende Wärmefarbbeständigkeit der Reaktionsansätze zu verfärbten, meist gelblichen bis gelben Polyisocyanaten.

Diese Gelbfärbung stört, insbesondere bei der Anwendung auf dem Lackgebiet, häufig die gleichmäßige Farbgebung, besonders bei pigmentierten Metalleffektlacken.

Wie nun überraschenderweise gefunden wurde, ist es möglich, diese Nachteile dadurch auszuschalten, daß man die zur Herstellung der Lackpolyisocyanate eingesetzten einfachen Diisocyanate einer Vorbehandlung mit Verbindungen, welche

$$-NH-\underset{\underset{\displaystyle O}{\|}}{C}-\text{Gruppen}$$

aufweisen, unterzieht.

Die US-PS 4 388 245 beschreibt zwar bereits ein Verfahren, bei welchem modifizierte Polyisocyanate, insbesondere Biuretgruppen aufweisende Polyisocyanate mit monomeren Diisocyanaten unter anschließender destillativer Entfernung des monomeren Diisocyanats erhitzt werden, jedoch kann das Verfahren dieser Vorveröffentlichung mit dem nachstehend näher beschriebenen erfindungsgemäßen Verfahren in keinem näheren Zusammenhang gebracht werden, da es beim Verfahren der genannten US-PS einzig und allein darum geht, höherfunktionellen Komponenten in den modifizierten Polyisocyanaten durch Hitzebehandlung mit monomeren Diisocyanaten in modifizierte Polyisocyanate einer verminderten Funktionalität zu überführen. Irgendwelche Mitteilungen bezüglich der Qualität des dann vom modifizierten Polyisocyanat abdestillierten monomeren Diisocyanats werden in der Vorveröffentlichung nicht mitgeteilt. Ganz entsprechend der grundverschiedenen Aufgabenstellung werden beim Verfahren der Vorveröffentlichung auch ganz andere Mengenverhältnisse der Reaktionspartner eingesetzt, als dies erfindungsgemäß der Fall ist.

Auch bei den klassischen Verfahren der Herstellung von Biuretpolyisocyanaten (vgl. z. B. US-PS 3 903 127) werden bereits gebildete Biuretpolyisocyanate im weiteren Verlauf der Biuretisierungsreaktion in Gegenwart von im Überschuß vorliegenden monomeren Diisocyanaten erhitzt, worauf sich ein Abdestillieren des Diisocyanat-Überschusses anschließt. Hier gilt jedoch bezüglich der Mengenverhältnisse die bereits im Zusammenhang mit US-PS 4 388 245 gemachte Aussage, daß nämlich von einer Erhitzung von monomerem Diisocyanat mit lediglich 0,1 bis 3 Gew.-% eines Biuretpolyisocyanats ebensowenig die Rede ist wie von der erfindungsgemäßen Aufgabenstellung, d.h. der erfindungsgemäß angestrebten Verbesserung der Wärmefarbbeständigkeit des monomeren Diisocyanats.

Gegenstand der Erfindung ist ein Verfahren zur Herstellung von wärmefarbbeständigen aliphatischen und/oder cycloaliphatischen Diisocyanaten, dadurch gekennzeichnet, daß man ein technisches Diisocyanat mit aliphatisch und/oder cycloaliphatisch gebundenen Isocyanatgruppen in Gegenwart von 0,1 bis 3 Gew.-%, bezogen auf das Diisocyanat, mindestens einer in dem Diisocyanat löslichen Verbindung, welche mindestens 3 Gew.-% an Struktureinheiten der Formel

$$-NH-\underset{\underset{\displaystyle O}{\|}}{C}-$$

aufweist, während eines Zeitraums von bis zu 5 Stunden auf eine Temperatur von 100 bis 220°C erhitzt und anschließend das so behandelte Diisocyanat destillativ reindarstellt.

Gegenstand der Erfindung ist auch die Verwendung der erfindungsgemäßen Verfahrensprodukte zur Herstellung von farbverbesserten, modifizierten Polyisocyanaten.

Für das erfindungsgemäße Verfahren geeignete Ausgangsdiisocyanate sind beispielsweise solche der Formel

Q $(NCO)_2$

in welcher

Q für einen aliphatischen Kohlenwasserstoffrest mit 2 bis 18, vorzugsweise 6 bis 10 Kohlenstoffatomen, einen cycloaliphatischen Kohlenwasserstoffrest mit 4 bis 15, vorzugsweise 5 bis 10 Kohlenstoffatomen oder einen aliphatisch-cycloaliphatischen Kohlenwasserstoffrest mit 6 bis 15, vorzugsweise 7 bis 12 Kohlenstoffatomen steht.

Die Begriffe "aliphatisch", "cycloaliphatisch" und "aliphatisch-cycloaliphatisch" beziehen sich auf die Art der mit den Isocyanatgruppen verknüpften Kohlenstoffatome der Kohlenwasserstoffreste.

Typische Beispiele geeigneter Ausgangsdiisocyanate sind aliphatische Diisocyanate wie 1,2-Diisocyanatoethan, 1,4-Diisocyanatobutan, 1,6-Diisocyanatohexan, 1,10-Diisocyanatodecan oder 1,18-Diisocyanato-octadecan, cycloaliphatische Diisocyanate wie 1,3-Diisocyanato-cyclobutan, 1,4-Diisocyanatocyclohexan, 4,4'-Diisocyanato-dicyclohexylmethan, dessen Gemische mit 2,4'-Diisocyanato-dicyclohexylmethan, 3,4'-Diisocyanato-4-methyldicyclohexylmethan oder aliphatisch-cycloaliphatische Diisocyanate wie 1-Isocyanato-3-isocyanatopropyl-1,3-dimethyl-cyclopentan oder 1-Isocyanato-3,3,5-trimethyl-5-isocyanatomethyl-cyclohexan (Isophorondiisocyanat, abgekürzt: IPDI). 1,6-Diiso-cyanatohexan und Isophorondiisocyanat, sind die bevorzugten beim erfindungsgemäßen Verfahren einzusetzenden Ausgangsisocyanate. 1-6-Diisocyanatohexan ist besonders bevorzugt. Grundsätzlich ist es auch möglich, beim erfindungsgemäßen Verfahren Gemische der beispielhaft genannten Diisocyanate einzusetzen, obwohl dies wenig zweckmäßig und daher weniger bevorzugt ist.

Die erfindungsgemäß zu behandelnden Diisocyanate werden beim erfindungsgemäßen Verfahren in "technischer Reinheit" eingesetzt. Diese bedeutet, daß es sich im allgemeinen bei den Ausgangsdiisocyanaten des erfindungsgemäßen Verfahrens um die destillativ aufgearbeiteten Phosgenierungsprodukte der den Diisocyanaten zugrundeliegenden Diamine handelt. Die Ausgangsdiisocyanate weisen im allgemeinen einen Reinheitsgrad von mindestens 99 % auf.

Beim erfindungsgemäßen Verfahren werden die Ausgangsdiisocyanate in Gegenwart von Verbindungen (nachstehend "Hilfsmittel" genannt), welche Struktureinheiten der Formel

$$-NH-\underset{\underset{O}{\|}}{C}-$$

aufweisen, während eines Zeitraums von bis zu 5, vorzugsweise von 1 bis 2 Stunden auf 100 bis 220°C, vorzugsweise 150 bis 190°C erhitzt. Voraussetzung für die Eignung der Hilfsmittel ist ihre Löslichkeit in den erfindungsgemäß zu behandelnden Ausgangsdiisocyanaten. Die Struktureinheit der Formel

$$-NH-\underset{\underset{O}{\|}}{C}-$$

kann in den Hilfsmitteln Teil einer Allophanat-, Biuret-, Harnstoff oder Urethangruppe sein. Dies bedeutet, daß es sich bei den Hilfsmitteln um Allophanat-, Biuret-, Harnstoff- und/oder Urethangruppen aufweisende Verbindungen handelt. In den Hilfsmitteln liegen die genannten Gruppierungen in solchen Mengen vor, die einen Gehalt der Hilfsmittel an NH-CO-Gruppen von mindestens 3 Gew.-% entsprechen. Die Hilfsmittel werden vorzugsweise dem zu behandelnden Diisocyanat zugesetzt; es ist jedoch auch möglich, die Hilfsmittel in situ dadurch herzustellen, daß man die Ausgangsdiisocyanate mit einer alkoholische Hydroxylgruppen und/oder primäre bzw. sekundäre Aminogruppen aufweisenden Verbindung versetzt, so daß ein Teil der Isocyanatgruppen des Ausgangsdiisocyanats mit der zugesetzten Verbindung unter Ausbildung von Struktureinheiten der genannten Art abreagiert. Die Menge der Hilfsmittel bzw. der zwecks Bildung der Hilfsmittel in situ zugesetzten Verbindung wird beim erfindungsgemäßen Verfahren so bemessen, daß in dem resultierenden Gemisch, bezogen auf freies Ausgangsdiisocyanat, 0,1 bis 3, vorzugsweise 0,5 bis 2 Gew.-% der zugesetzten bzw. in situ gebildeten Hilfsmittel vorliegen.

Geeignete, den Ausgangsdiisocyanaten zuzusetzende Hilfsmittel sind beispielsweise

(i) in den Ausgangsdiisocyanaten lösliche Harnstoffe, wie z. B. Harnstoffgruppen aufweisende Umsetzungsprodukte von organischen Isocyanaten, insbesondere von Diisocyanaten der erfindungsgemäß als Ausgangsdiisocyanate geeigneten Art mit primären oder sekundären Aminen wie 3,3,5-Trimethylcyclohexylamin, Dicyclohexylamin, N-Methylcyclohexylamin, 3-Amino-1,2,4-triazol, Stearylamin, Methyloctadecylamin, Dodecylamin und 1-Dodecyl-2,4-diamino-benzol,

(ii) in den Ausgangsdiisocyanaten lösliche Biurete wie z. B. Tris-(isocyanatohexyl)-biuret oder sein Gemisch mit seinen höheren Homologen bzw. analoge Biuretpolyisocyanate auf Basis anderer Diisocyanate der erfindungsgemäß als Ausgangsmaterial einzusetzenden Art;

(iii) in den Ausgangsdiisocyanaten lösliche, Urethangruppen aufweisende Verbindungen, beispielsweise Urethangruppen aufweisende Umsetzungsprodukte von organischen Isocyanaten, insbesondere von Diisocyanaten der erfindungsgemäß als Ausgangsmaterial einzusetzenden Art mit ein- und/oder mehrwertigen Alkoholen des Molekulargewichtsbereichs 32 bis 2000, vorzugsweise 32 bis 200 wie z. B. Methanol, n-Butanol, n-Hexanol, Ethylenglykol, Propylenglykol, Diethylenglykol, Triethylenglykol, Dipropylenglykol, Tripropylenglykol und/oder Oligo- bzw. Polyesterpolyole auf Basis von Dicarbonsäuren wie Adipinsäure und Diolen der zuletzt beispielhaft genannten Art;

(IV) Allophanate, wie sie durch Erhitzen von überschüssigen Mengen an organischen Isocyanaten, insbesondere von Diisocyanaten der erfindungsgemäß als Ausgangsmaterial einzusetzenden Art mit Urethanen der unter (iii) genannten Art erhalten werden können.

Besonders bevorzugt beim erfindungsgemäßen Verfahren einzusetzende Hilfsmittel sind Biuretgruppen aufweisende Hilfsmittel, insbesondere die oben unter (ii) beispielhaft genannten Biuretpolyisocyanate.

Geeignete Verbindungen, die mit den erfindungsgemäß zu behandelnden Diisocyanaten in situ unter Bildung geeigneter erfindungsgemäßer Hilfsmittel abreagieren sind beispielsweise tert. Butanol, Dodecylamin, 1-Dodecyl-2,4-diamino-benzol, 3-Amino-1,2,4-triazol, oder alkoholische Verbindungen der oben unter (iii) beispielhaft genannten Art, wobei jedoch in orientierenden Vorversuchen jeweils ermittelt werden muß, ob das sich in situ bildende Hilfsmittel in dem jeweils zu behandelnden Ausgangsdiisocyanat löslich ist.

Nach der erfindungsgemäßen Hitzebehandlung wird das im Überschuß vorliegende Ausgangsdiisocyanat destillativ, vorzugsweise durch Vakuumdestillation in geeigneten Destillationsapparaturen zurückgewonnen.

Die so behandelten Diisocyanate zeichnen sich gegenüber den entsprechenden nicht erfindungsgemäß behandelten Diisocyanaten durch eine erhöhte Wärmefarbbeständigkeit aus. Dies ist insbesondere bei der Weiterverarbeitung der Diisocyanate zu höherfunktionellen Lackpolyisocyanaten von Bedeutung, da die resultierenden Lackpolyisocyanate im Unterschied zu entsprechenden Lackpolyisocyanaten auf Basis von nicht erfindungsgemäß behandelten Diisocyanaten eine geringere Eigenfarbe aufweisen. Bei der erfindungsgemäßen Verwendung der erfindungsgemäßen Verfahrensprodukte zur Herstellung von Lackpolyisocyanaten, d.h. von modifizierten Polyisocyanaten, insbesondere von Isocyanurat- oder Biuretmodifizierten Polyisocyanaten wird nach den an sich bekannten Verfahren des Standes der Technik vorgegangen. Die Herstellung von Isocyanuratgruppen aufweisenden Polyisocyanaten kann beispielsweise gemäß DE-OS 3 033 860, DE-OS 3 100 262, DE-OS 3 100 263, US-PS 4 324 879 oder US-PS 4 288 586 erfolgen. Die Herstellung von Biuretpolyisocyanaten kann beispielsweise gemäß US-PS 3 124 605, US-PS 3 358 010 oder EP-PS 35 05 erfolgen.

Die in den nachfolgenden Beispielen gemachten Prozentangaben beziehen sich, soweit nichts anderslautendes angemerkt, auf Gewichtsprozente.

**Beispiel 1**

2800 g 1-6-Diisocyanatohexan (HDI) werden mit 12 g 3-Amino-1,2,4-triazol gemischt und auf 185°C aufgeheizt. Bei 125-130°C geht das Aminotriazol unter Reaktion in Lösung. Der Ansatz wird 1,5 Stunden bei 185°C unter $N_2$ gerührt; anschließend wird bei 165°C und 20 mm Hg das HDI abdestilliert. 2016 g (12 Mol) des auf diese Weise vorbehandelten HDI und 148 g (2 Mol) tert. Butanol werden gemischt und 10 Minuten bei Raumtemperatur unter $N_2$ gerührt. Anschließend wurde der Stickstoffstrom abgestellt und die Mischung auf 185°C aufgeheizt. Über eine angeschlossene Gasuhr wird das entwickelte Volumen an Isobutylen und $CO_2$ gemessen.

| Zeit (Min) | Temperatur (°C) | Volumen (ltr) |
|---|---|---|
| 0 | 185 | 20,5 |
| 5 | 185 | 29,0 |
| 10 | 185 | 36,3 |
| 15 | 185 | 42,3 |
| 20 | 185 | 47,7 |
| 25 | 185 | 53,4 |
| 30 | 185 | 58,4 |
| 35 | 185 | 62,3 |
| 40 | 185 | 66,5 |
| 50 | 185 | 73,4 |
| 60 | 185 | 79,2 |
| 70 | 185 | 83,5 |
| 80 | 185 | 87,1 |
| 90 | 185 | 89,3 |

Das Rohprodukt wird abgekühlt und anschließend 2 x bei 160°C am Dünnschichtverdampfer destilliert. Man erhält auf diese Weise ein klares, leicht gelbes Produkt mit folgenden Kenndaten:

| | |
|---|---|
| % NCO | 22,01 |
| $\eta$25°C | 6288 mPas |
| HAZEN Farbzahl | 100 Apha (gemäß DIN 53 409) |
| Monomerengehalt | 0,5 % freies HDI |

**Beispiel 2**

3000 g HDI werden mit 30 g eines Biuretpolyisocyanats auf Basis von 1,6-Diisocyanatothexan mit einem NCO-Gehalt von 22,1 %, welches zu ca. 30 % aus Tris-(isocyanatohexyl)-biuret und zum Rest aus dessen höheren Homologen besteht, gemischt und während 1,5 Stunden bei 185°C unter $N_2$ gerührt; anschließend wird bei 165°C und 20 mm Hg das HDI abdestilliert.

2520 g (15 Mol) des auf diese Weise vorbehandelten HDI und 185 g (2)5) tert. Butanol werden gemischt und 10 Minuten bei Raumtemperatur unter Stickstoff gerührt. Anschließend wird der Stickstoffstrom abgestellt und die Mischung auf 185°C aufgeheizt. Über eine angeschlossene Gasuhr wird das entwickelte Volumen an Isobutylen und $CO_2$ gemessen.

| Zeit (Min) | Temperatur (°C) | Volumen (ltr) |
|---|---|---|
| 0 | 185 | 7,1 |
| 5 | 185 | 15,7 |
| 10 | 185 | 24,7 |
| 15 | 185 | 32,8 |
| 20 | 185 | 40,5 |
| 25 | 185 | 47,5 |
| 30 | 185 | 53,5 |
| 35 | 185 | 59,4 |
| 40 | 185 | 64,3 |
| 50 | 185 | 72,6 |
| 60 | 185 | 81,5 |
| 70 | 185 | 88,9 |
| 80 | 185 | 94,4 |
| 90 | 185 | 99,0 |
| 100 | 185 | 107,0 |
| 110 | 185 | 109,0 |
| 120 | 185 | 111,5 |
| 120 | 185 | 113,0 |

Das Rohprodukt wird abgekühlt und anschließend 2 x bei 160°C 0,2 bis 0,4 mm Hg am Dünnschichtverdampfer destilliert.

Man erhält auf diese Weise ein klares, schwach gelbes Produkt mit folgenden Kenndaten:

| | |
|---|---|
| % NCO | 21,8 |
| η 25°C | 9421 mPas |
| HAZEN Farbzahl | 90 Apha (gemäß DIN 53 409) |
| Monomerengehalt | 0,21 % freies HDI |

**Vergleichsbeispiel**

3000 g technisches HDI werden während 1,5 Stunden bei 185°C unter Stickstoff gerührt; anschließend wird bei 165°C und 20 mm Hg das HDI abdestilliert.

2520 g (15 Mol) des auf diese Weise vorbehandelten HDI und 185 g (2,5 Mol) tert. Butanol werden gemischt und 10 Minuten bei Raumtemperatur unter Stickstoff gerührt. Anschließend wird der Stickstoffstrom abgestellt und die Mischung auf 185°C aufgeheizt. Über eine angeschlossene Gasuhr wird das entwickelte Volumen an Isobutylen und $CO_2$ gemessen.

| Zeit (Min) | Temperatur (°C) | Volumen (ltr) |
|---|---|---|
| 0 | 185 | 12,5 |
| 5 | 185 | 23,1 |
| 10 | 185 | 33,8 |
| 15 | 185 | 42,5 |
| 20 | 185 | 50,5 |
| 25 | 185 | 57,5 |
| 30 | 185 | 64,9 |
| 35 | 185 | 71,2 |
| 40 | 185 | 76,5 |
| 50 | 185 | 86,5 |
| 60 | 185 | 95,1 |
| 70 | 185 | 101,8 |
| 80 | 185 | 107,4 |
| 90 | 185 | 111,6 |
| 100 | 185 | 112,1 |

Das Rohprodukt wird abgekühlt und anschließend 2 x bei 160°C 0,2 bis 0,4 mm Hg am Dünnschichtverdampfer destilliert.

Man erhält auf diese Weise ein klares, gelbes Produkt mit folgenden Kenndaten:

| | |
|---|---|
| % NCO | 22,44 |
| η25°C | 6851 mPas |
| HAZEN Farbzahl | 520 Apha (gemäß DIN 53 409) |
| Monomerengehalt | 0,16 % freies HDI |

**Patentansprüche**

1. Verfahren zur Herstellung von wärmefarbbeständigen aliphatischen und/oder cycloaliphatischen Diisocyanaten, dadurch gekennzeichnet, daß man ein technisches Diisocyanat mit aliphatisch und/oder cycloaliphatisch gebundenen Isocyanatgruppen in Gegenwart von 0,1 bis 3 Gew.-%, bezogen auf das Diisocyanat, mindestens einer in dem Diisocyanat löslichen Verbindung, welche mindestens 3-Gew.-% an Struktureinheiten der Formel

$$-NH-\underset{\underset{O}{\|}}{C}-$$

aufweist, während eines Zeitraums von bis zu 5 Stunden auf eine Temperatur von 100 bis 220°C erhitzt und anschließend das so behandelte Diisocyanat-destillativ reindarstellt.

2. Verfahren gemäß Anspruch 1, dadurch gekennzeichnet, daß man als Verbindungen mit Struktureinheiten der Formel

$$-NH-\underset{\underset{O}{\|}}{C}-$$

Allophanat-, Biuret-, Harnstoff- oder Urethangruppen aufweisende Umsetzungsprodukte von organischen Polyisocyanaten mit aliphatisch und/oder cycloaliphatisch gebundenen Isocyanatgruppen verwendet.

3. Verfahren gemäß Anspruch 1 und 2, dadurch gekennzeichnet, daß man als Verbindung mit Struktureinheiten der Formel

$$-NH-\underset{\underset{O}{\|}}{C}-$$

Biuretgruppen aufweisende Polyisocyanate verwendet.

4. Verfahren gemäß Anspruch 1 bis 3, dadurch gekennzeichnet, daß man die Verbindung mit Struktureinheiten der Formel

$$-NH-\underset{\underset{O}{\|}}{C}-$$

in situ dadurch herstellt, daß man das zu behandelnde technische Diisocyanat mit einer alkoholische Hydroxylgruppe und/oder primäre oder sekundäre Aminogruppen aufweisenden Verbindung versetzt, so daß ein Teil der Isocyanatgruppen des technischen Diisocyanats mit der zugesetzten Verbindung unter Allophanat-, Biuret-, Harnstoff- oder Urethangruppen abreagiert.

5. Verwendung der gemäß Anspruch 1 bis 4 erhaltenen Diisocyanate einer verbesserten Wärmefarbbeständigkeit zur Herstellung von farbverbesserten, modifizierten Polyisocyanaten.

## Claims

1. Process for the preparation of aliphatic and/or cycloaliphatic diisocyanates which are colour stable in the heat, characterised in that a commercial diisocyanate containing aliphatically and/or cycloaliphatically bound isocyanate groups is heated to a temperature of from 100°C to 220°C for a period of up to 5 hours in the presence of from 0.1 to 3 % by weight, based on the diisocyanate, of at least one compound which is soluble in the diisocyanate and which contains at least 3 % by weight of structural units of the formula

$$-NH-\underset{\underset{O}{\|}}{C}-$$

,and the diisocyanate which has been treated by this method is then obtained in the pure form by distillation.

2. Process according to Claim 1, characterised in that the compounds used as compounds containing structural units of the formula

$$-NH-\underset{\underset{O}{\|}}{C}-$$

are reaction products obtained by the reaction of organic polyisocyanates with aliphatically and/or cycloaliphatically bound isocyanate groups and containing allophanate, biuret, urea or urethane groups.

3. Process according to Claims 1 to 2, characterised in that polyisocyanates containing biuret groups are used as compound containing structural units of the formula

-NH-C-
||
O

.

4. Process according to Claims 1 to 3, characterised in that the compound containing structural units of the formula

-NH-C-
||
O

is prepared in situ by adding a compound containing alcoholic hydroxyl groups and/or primary or secondary amino groups to the commercial diisocyanate to be treated so that a proportion of the isocyanate groups of the commercial diisocyanate reacts with the added compound to form allophanate, biuret, urea or urethane groups.

5. Use of the diisocyanates with improved colour stability in the heat obtained according to Claims 1 to 4 for the preparation of colour improved, modified polyisocyanates.

**Revendications**

1. Procédé de production de diisocyanates aliphatiques et/ou cycloaliphatiques de couleur stable à la chaleur, caractérisé en ce qu'on chauffe pendant une durée allant jusqu'à 5 heures à une température de 100 à 220°C un diisocyanate technique à groupes isocyanato en liaison aliphatique et/ou cycloaliphatique en présence de 0,1 à 3 % en poids, par rapport au diisocyanate, d'au moins un composé soluble dans le diisocyanate, qui présente au moins 3 % en poids de motifs structuraux de formule

-NH-C-
||
O

,

puis on purifie par distillation le diisocyanate ainsi traité.

2. Procédé suivant la revendication 1, caractérisé en ce qu'on utilise comme composés à motifs structuraux de formule

-NH-C-
||
O

des produits de réaction, porteurs de groupes allophanate, biuret, urée ou uréthanne, de polyisocyanates organiques porteurs de groupes isocyanato en liaison aliphatique et/ou cycloaliphatique.

3. Procédé suivant les revendications 1 et 2, caractérisé en ce qu'on utilise comme composé à motifs structuraux de formule

-NH-C-
||
O

des polyisocyanates porteurs de groupes biuret.

4. Procédé suivant les revendications 1 à 3, caractérisé en ce qu'on prépare le composé à motifs structuraux de formule

-NH-C-
||
O

in situ en ajoutant au diisocyanate technique à traiter un composé porteur d'un groupe hydroxyle alcoolique et/ou porteur de groupes amino primaires ou secondaires de manière qu'une partie des groupes isocyanato du diisocyanate technique réagissent avec le composé ajouté avec formation de groupes allophanate, biuret, urée ou uréthanne.

5. Utilisation des diisocyanates obtenus conformément aux revendications 1 à 4, dont la stabilité thermique de la couleur est améliorée, pour la production de polyisocyanates modifiés, de couleur améliorée.